# EUROPEAN PATENT SPECIFICATION

(11) **EP 0 319 267 B1**
(45) Date of publication and mention of the grant of the patent: **21.04.1993**
(21) Application number: 88311340.9
(22) Date of filing: 30.11.1988
(51) Int. Cl.: A61M 5/14

(54) **Fluid delivery control and monitoring apparatus**
Volumenstromsteuerungs- und Überwachungsgerät
Dispositif de commande et de surveillance de débit de fluide

(30) Priority: 04.12.1987 US 128973
(43) Date of publication of application: 07.06.1989
(73) Proprietor: Pacesetter Infusion Limited doing business as Minimed Technologies, Sylmar California 91342 (US)
(72) Inventor: Slate, John B., Studio City California 91604 (US); Henke, James L., Simi Valley California 93063 (US)
(74) Representative: Rees, David Christopher

(56) References cited:
- EP-A- 0 183 400
- GB-A- 2 126 666
- US-A- 4 345 483
- US-A- 4 601 702
- US-A- 4 617 014
- MEDICAL PROGRESS TRHROUGH TECHNOLOGY, vol. 8, no. 1, 1980, pages 49-56,Springer-Verlag 1980, West-Berlin, DE; G.A. CARLSON et al.: "A portable insulininfusion system with a rotary solenoid-driven peristaltic pump"

## Description

The present invention relates generally to an electromechanical system for continuously delivering fluid. The invention is particularly applicable to a fluid delivery control and monitoring apparatus used in a medication infusion system.

There are two known major techniques available for delivering drugs to a patient when the drugs cannot be orally administered. The first technique is to inject the drug into the patient with a syringe and needle to deliver an appreciable dose at relatively infrequent intervals. This technique is not always satisfactory, particularly when the drug being injected is potentially lethal, possibly has undesirable side effects when given in a large dosage, or must be delivered more or less continuously to arrive at a desired therapeutic result. This technique leaves much to be desired. The risks of overdosage or harmful side effects may be reduced by giving smaller injections at more frequent intervals, an inconvenient and not altogether satisfactory alternative.

The need for delivering a drug more or less continuously gives rise to the second technique, which involves a continuous delivery of medication to the patient, typically through an intravenous drip. Medication may also be administered using an intravenous (IV) system with an injection into a complicated and cumbersome interconnection of IV tubes, hoses and other components. Drop counters are used to measure the amount of fluid delivered, and medications are often delivered in a larger dose through injection into the IV lines, with the medication being somewhat diluted by the fluid.

A relatively recent alternative to these two techniques of administering medication to a patient is the medication infusion pump. A valuable and much needed development, the medication infusion pump can be used to administer drugs to a patient in small, carefully measured doses at frequent intervals or, with some devices, slowly but uninterruptedly. A therapeutic regimen with an infusion pump can be controlled electronically to administer precisely measured quantities of a drug at precisely planned intervals to give a gradual infusion of medication into the patient. The infusion pump makes possible a closer approximation to the natural maintenance of biochemical balances in the body because of its ability to deliver a repetitive small dose.

Disposability is an important consideration in the design of medication infusion systems. Parts of the system through which medication is pumped must be sterile, so that in most applications some of the equipment is used and then discarded. The disposable parts are typically replaced at regular intervals, typically on a daily basis. Disposability of the fluid pump portion of the infusion device is a highly desirable feature. It would be very convenient to design a fluid pump in the form of an attachable cassette of economical design which could easily be installed onto a main pumping unit. A cassette which uses a small number of parts, is easily mass producible, and is capable of delivering liquid medication or other therapeutic fluids with a high degree of precision is described in our European Application No. 88311354.0 (EP-A-0319278).

The disposable cassette which is referred to above includes a fluid pump affording a high degree of accuracy in fluid delivery, with the degree of accuracy being maintained throughout the life of the product. The cassette also provides means for conveniently and easily priming the pump, and includes a bubble trap to prevent the frequent shutdowns and alarms which are a problem with presently available pumps. The cassette also includes additional devices such as pressure sensing means and bubble detecting means which in conventional medication infusion systems constitute separate assemblies.

A fluid monitoring and control system for use with disposable cassettes is needed to ensure accurate and safe delivery of therapeutic fluids. The design of such a system requires careful attention to factors involved in the accuracy of fluid delivery, and instrument monitoring functions are necessary to ensure safe operation of the system.

US-A-4617014 (Cannon et al) discloses an intravenous infusion device combining both a flow control device and pump for the delivery of solutions through an IV tube.

A pressure sensing strain gauge is mounted on the tube. This is operably connected with a processor which creates an alarm condition when the device is pumping and the downstream pressure reaches a threshold and when the device is controlling a flow and there is no pressure differential across the gauge. The pump is driven by a stepper motor controlled by the processor and provided with a shaft encoder giving motor position information.

Thus fluid delivery, monitoring and control apparatus is known for use in a medication infusion system which comprises: drive means for actuating pistons and valves in the fluid pathway and monitoring means, having inputs from sensors in the drive means; control means having inputs from the monitoring means, from the drive means, and from command signals, and an output connected to control the drive means; and encoding means coupled to the drive means and arranged to provide signals indicative of the position of the drive means.

There has been a long-felt but unresolved need for the development of a medication infusion management system that can be used for patient care in both hospitals and home health care applications. A desirable system would provide a reliable and improved product for current applications to encourage the use of new therapeutic techniques, reduce the cost of hospitalisation by improving care and decreasing labour and inventory costs, and would be versatile enough to allow intra-arterial and subcutaneous infusions. Primary requirements of such a system would be volumetric accuracy, state-of-the-art safety functions, and a capacity for independently controlling more than one pumping channel, each with a separate line to the patient.

Ideally the pump of the medication infusion system would be substantially smaller and lighter than current hospital pumps while at the same time incorporating multiple pumping channels. Together with the possibility of extended battery-powered operation, these features would make a device that is very well suited to ambulatory care, intensive care, emergency care, and operating room use.

A system with the capacity for multiple pumping channels, a variety of disposable configurations, and a library of software functions could combine the capabilities of several currently available devices into one single unit. For example, the need in a hospital for separate syringe pumps, PCA pumps, neonatal pumps, general purpose pumps, and computer communications pumps could be eliminated in favour of our system that could satisfy the requirements for all these devices.

The necessity for cost containment is recognised in the health care industry. Costs can be broken up into three categories: material, labour, and maintenance. With intravenous infusion devices, a major consideration is the cost of disposables, since a large number are required per year. A medication infusion system that keeps down the cost of disposables, is easy to set up, simple to use, and highly reliable would be a great boon to the health care field.

The present invention is characterised in that the pistons and valves are mounted in a disposable cassette, the monitoring means also having inputs from sensors in the cassette; the encoding means are also arranged to provide rate of movement signals from the drive means; the monitoring means include means for ascertaining fluid flow rate from the rate of movement signals and from cassette indicia, indicating piston stroke volume, and for generating feedback signals indicative of sensed flow rate; and in that the control means include means for receiving rate command signals, defining a desired rate of fluid flow through an associated cassette, and means for combining the rate command signals with the feedback signals to develop signals for controlling the drive means.

In a preferred embodiment fluid pumping is accomplished for multiple channels by independently controlling DC motors in the drive mechanism, each of which is coupled to two valves and a reciprocating piston for an individual pumping channel in the cassette. The motor is geared down to provide an appropriate maximum speed and torque. A cam can sequentially actuate an inlet valve, piston, and outlet valve in the cassette. Initially the cam is at a "home" position (180°) with the inlet valve open and outlet valve closed, with the pump cylinder ready to be filled. Starting at the home position, the motor rotates the cam at maximum speed, backing the piston to the top of its stroke and thus rapidly filling the cylinder. Next the inlet valve is closed and the outlet valve is opened; having one valve always closed in this manner prevents free-flow. A range of fluid delivery rates is achieved by periodically sending pulses to the motor. At the end of the piston delivery stroke, the motor operates at maximum speed to close the outlet valve and open the inlet valve, thus completing the pumping cycle.

Preferably, the average flow output of the pump is maintained near the set point rate by a digital feedback controller which uses closed-loop feedback control to provide accurate regulation. This is desirable because of differences in load parameters created by drive and cassette friction, inertia, and fluid back pressure. At regular time intervals, the controller computes the motor voltage pulse width based upon feedback information from an encoder. Two different pulse amplitude levels are used to provide a range of average flow rates from, for example, 0.1 to 999 ml/hr (millilitres per hour), using an 80 µl stroke volume. The drive speed of the motor can be correlated with fluid flow to arrive at a flow rate calibration. Errors in flow rate are minimised by regulating the motor speed accurately.

Preferably, a drive shaft encoder supplies information necessary for operation of the pump with respect to: 1) a home position; 2) a delivery stroke marker; 3) incremental delivery markers; and 4) a brake marker at the end of the refill cycle. For very small flow rates it is desirable to be able to stop the motor at the end of the refill cycle. This is done by shorting the motor windings during a braking period.

Preferably, a fluid delivery monitoring system detects conditions which could endanger the patient, such as 1) loss of fluid flow due to an occlusion somewhere in the system; 2) inaccuracy in the volume of fluid delivered greater than a specified amount; 3) infusion of significant amounts of air into the patient; 4) lack of proper latching of the cassette into place and 5) instrument malfunction.

In another preferred embodiment three pumping channels are operated independently. The pumping arrangement is modular to simplify construction, testing and repair and to allow the possibility of providing one- and two-channel versions with common parts. Each pump module comprises a bottom support structure, a drive mechanism, a motor, an anti-backdrive clutch (brake) and an encoder.

A specific embodiment of the present invention will now be described by way of example with reference to the accompanying drawings in which:
Figure 1 is a block diagram of a fluid delivery monitoring and control system according to the invention;
Figure 2 is a block diagram of the fluid delivery system;
Figure 3 is a diagram illustrating the different phases of the pumping cycle;
Figure 4 is a top view of an encoder disc;
Figure 5 is a block diagram of a delivery rate control system;

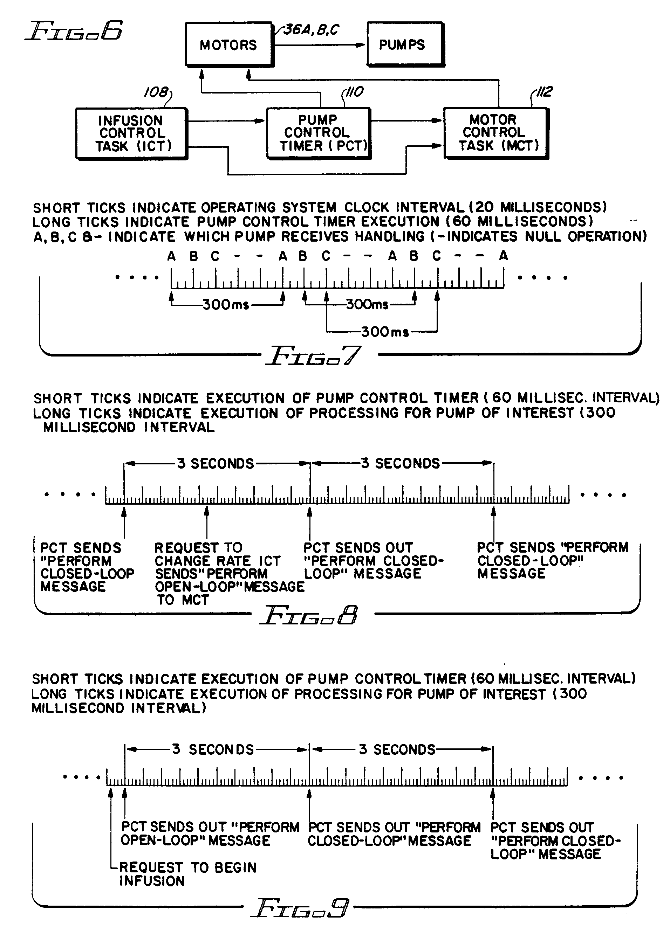

Figure 5 is a block diagram of the field delivery control procedures;
Figure 7 is a timing diagram for the pump controller;
Figure 8 is a timing diagram for starting infusion; and
Figure 9 is a timing diagram for a pumping rate change.

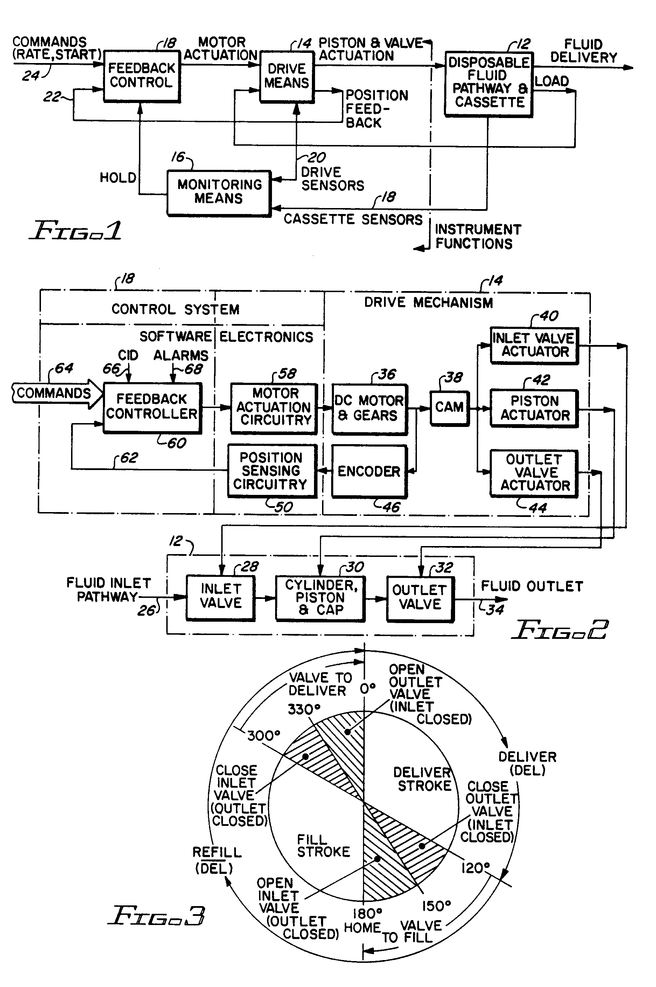

Figure 1 shows the overall organisation of a fluid delivery monitoring and control apparatus 10 according to the present invention for use in a medication infusion system employing a disposable fluid pathway and cassette 12. The fluid delivery monitoring and control apparatus 10 comprises three major functional blocks: drive means 14, monitoring means 16, and control means 18. The disposable fluid pathway and cassette 12 is described in detail in our European Patent Application No. 88311354.0 (EP-A-0319278). In brief, the disposable cassette has only seven components and utilises a highly accurate and reliable piston-type fluid pump which employs an active valve design of great accuracy and precision despite its simplicity. A bubble trap is included in the cassette for removing air bubbles which may be introduced into the system by the fluid supply apparatus. The cassette has standard Luer fittings on inlet and outlet tubes. In the preferred embodiment, the cassette also includes both a pressure diaphragm for enabling pressure sensing of the outlet line, and a bubble detector for detecting bubbles in excess of an acceptable size in the fluid supply to a patient.

The drive means 14 actuate one or more pumps contained in the disposable fluid pathway and cassette 12 which effect the delivery of fluid to a patient. The outputs of cassette sensors in the cassette 12 are sent to the monitoring means 16 via interconnection path 19. Information from drive sensors in the drive means 14 are communicated to the monitoring means 16 via interconnection 20. Position feedback information is sent from the drive means 14 to the control means 18 by way of path 22. The control means 18 actuate the drive means 14 in response to various commands on an input line 24. The control means 18 are also responsive to the monitoring means 16.

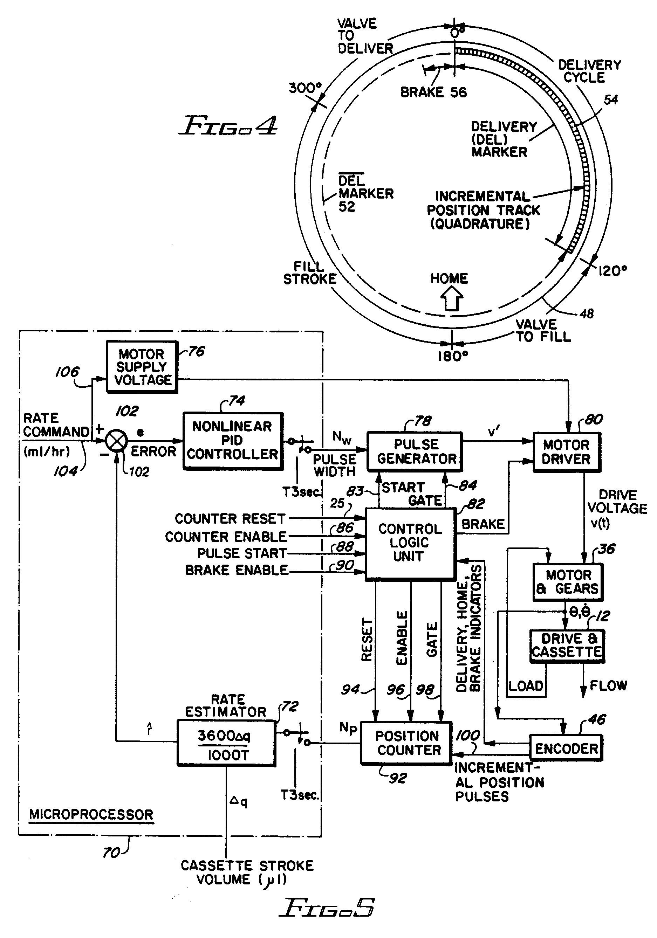

Further details of the drive means 14 and the control means 18 are shown in Figure 2. The disposable fluid pathway and cassette 12 is shown in Figure 2 as having only a single pumping channel, but in general there will be two or more independent pumping channels. The single pumping channel shown in Figure 2 comprises a fluid inlet pathway 26 followed by an inlet valve 28; a cylinder, piston, and cap arrangement 30; an outlet valve 32; and a fluid outlet pathway 34.

The drive means 14 comprises an ironless core DC motor and gears 36 with a cam 38 on a geared-down output shaft. The cam 38 drives an inlet valve actuator 40, a piston actuator 42, and an outlet valve actuator 44, all of which are mechanically coupled to, respectively, the inlet valve 28, a piston in arrangement 30, and the outlet valve 32, all in cassette 12. The DC motor typically has a built-in gear reduction unit to reduce the output speed.

As described in our European Patent Application No. 88311354.0 (EP-A-0319278) the end of the motor having the output shaft is mounted onto the top of a drive module chassis at one side thereof with the output shaft extending through the drive module chassis. A drive pulley is mounted on the output shaft and is driven by the motor. A unidirectional bearing is mounted onto the top of the drive module chassis at the other side thereof. The unidirectional bearing supports a drive shaft for rotation therein; both ends of the drive shaft extend from the unidirectional bearing. The unidirectional bearing allows the drive shaft to rotate in one direction only; in the preferred embodiment, the rotation is clockwise when viewed from the top. The power module cam 38 is mounted on the bottom end of the drive shaft extending from the unidirectional bearing. A drive belt is mounted over the drive pulley and in the groove in the power module cam 38. The motor will thereby drive the power module cam 38 and the drive shaft.

An encoder 46 provides four types of information necessary for the operation of the pump in cassette 12, as illustrated in figure 3. The various segments of the pumping cycle are shown, consisting of a "home" position, a delivery stroke segment, a closed outlet valve segment, an open inlet valve segment, a fill stroke segment, a closed inlet valve segment, and an open outlet valve segment.

Figure 4 is a top view of an encoder disc 48 having fiducial marks on it which are sensed by a position sensor. The encoder disc 48 is fixedly mounted on the top end of the motor drive shaft and rotates with the drive shaft and the cam 38. The position sensor is fixedly mounted above the unidirectional bearing from which the output shaft of the gear reduction unit extends. The position sensor provides position feedback information to a position sensing circuit 50, as shown in Figure 3. The position sensor is also capable of direction sensing.

Referring to Figure 4, the fiducial marks sensed by the position sensor are translated into various electronic signals such as a delivery stroke marker, an incremental delivery marker, and a brake marker. The encoder disc 48 and cam 38 are aligned so that the "home" position is correct. The home position is used to place the cassette piston latch in a known location in order to ensure safe and easy loading of the cassette 12. A delivery marker 52 indicates whether the system is delivering fluid or refilling (which includes valve-to-valve fill, fill and valve-to-deliver). This information is required because the piston must be moved into the cylinder at a controlled rate during the delivery cycle, whereas refilling must be completed as fast as possible to minimise interruption of fluid delivery. The incremental delivery markers 54 are closely spaced along a track on the periphery of the encoder disc 48. The incremental delivery markers 54 are translated into feedback signals for accurate and precise regulation of fluid output flow. When small increments are needed for delivery, it is necessary to stop the motor at the end of the refill cycle. This is accomplished by shorting the motor windings during the interval of the brake marker 56.

Referring again to Figure 2, the control means 18 are seen to comprise motor actuation circuitry 58, the position-sensing circuit 50, and a feedback controller 60. The feedback controller 60 has a position sensing input 62, a command input 64, a cassette ID input 66, and an alarm input 68. The feedback controller 60 is realised in software. As shown in Figure 5, a microprocessor 70 comprises a rate estimator 72, a nonlinear controller 74, and a motor supply voltage unit 76.

The motor actuation circuitry 58 comprises a pulse generator 78, a motor driver 80, and a control logic unit 82. The pulse generator 78 is fed a pulse of appropriate width by the microprocessor 70 via a data bus. Once a pulse is loaded, the microprocessor 70 starts the pulse generator 78 through an input 83. The output of the pulse generator 78 is logic high for the duration of the pulse width, otherwise logic zero. The pulse generator 78 is gated by the control logic unit 82 via a gate input 84.

Delivery, home and brake indicators are sent to the control logic unit 82 from the encoder 46. Inputs to the control logic unit 82 from the microprocessor 70 include counter reset 25, counter enable 86, pulse start 88, and brake enable 90. The control logic unit 82 is connected to a position counter 92 via a reset input 94, an enable input 96, and a gate input 98. The encoder 46 sends incremental position pulses to the position counter 92 via an input 100.

The microprocessor 70 samples the output of the position counter 92 at selected intervals (e.g. three seconds) to determine the amount of fluid delivered. A value for the cassette stroke volume is fed into the rate estimator 72 and together with data from the position counter 92 is used to determine an estimated rate, rₑ, which is fed into a comparator 102. The other input to the comparator 102 is a rate command signal 104. The rate command signal 104 also enters the motor supply voltage unit 76 through a parallel connection 106. The input to the nonlinear controller 74 is the sampled error signal, e, which is the difference between the rate command, r_{c}, and the estimated rate, rₑ. The nonlinear controller 74 is an algorithm which adjusts the pulse width based on the error signal. The calculated pulse width count is then transferred to the motor pulse width generator 78.

Figure 6 is a block diagram of the software procedures making up the implementation of the motor control algorithm for an embodiment in which there are three independent pumping channels. The three sets of motors and their associated gearing 36a, 36b, and 36c are controlled by three distinct software components which co-operate to generate controlled fluid delivery by three pumps. These three software procedures are the infusion control task 108, the motor control task 110, and the pump control timer 112. These three software components will be referred to as the ICT, MCT, and PCT and are described in more detail below.

### Cassette Loading and Latching

A loading and latching procedure secures the cassette to the instrument which prepares the system for fluid delivery. To ensure accurate fluid delivery and monitoring, the cassette must be held securely to the face of the instrument and the piston must be held securely by the piston actuator. A cassette slide clamp latches the cassette to the instrument. A piston actuator latch clamps the piston during loading. Based on sensor inputs that monitor cassette placement and piston latching, the instrument provides a prompt to the operator on set up prior to starting an infusion. There is only one way to install the cassette, and it is easy to remove and re-install.

The loading and latching process does not allow any unintentional delivery of fluid to the patient. Before loading, the cassette slide clamp is closed to occlude the line, and this also opens the cassette hold-down latch. The fluid pathway must be occluded by an outlet valve before the cassette slide clamp is opened during cassette latching. The piston actuator is positioned at the beginning of the full cycle (home position) prior to loading the cassette. Any movement of the piston fills the cylinder and will not displace fluid to the patient. The microprocessor detects proper cassette placement and piston latching; if a fault occurs, the infusion is stopped and audio and visual alarm signals are produced.

### Sensing Proper Cassette Installation

The instrument electronically detects whether a cassette has been loaded and latched properly. Two sets of sensors do this: cassette ID sensors and a piston latch-in-place (LIP) sensor. If a valid ID code is detected and the piston latch is in place, it is assumed that the cassette is properly installed.

The cassette ID sensors identify the cassette characteristics such as stroke volume and detect whether a cassette has been installed. Three emitter/detector pairs located in the instrument sensor bay read the ID code moulded into the cassette body. A three-bit redundant code is used to specify the flow range of the cassette.

The piston latch-in-place sensor consists of an emitter/detector pair mounted in the bottom of the sensor module. This sensor detects whether the piston actuator latch has been closed. The LIP sensor is interfaced to the microprocessor by polling an I/O port. The output of the detector is transformed into a digital logic level by a comparator, and the port is polled at the slowest frequency that does not result in a significantly perceptible delay in the alarm response. The microprocessor periodically checks the latch sensors and issues a hardware fault alarm if a sensor is found to be faulty. The cassette ID code is read by the microprocessor after 1) a change in LIP state occurs, 2) after power is turned on, and 3) after START is pressed. The ID sensors are checked before reading to ensure that they are operating properly. After a cassette has been properly attached, an audio feedback signal is given to the user.

### Pump Control

Several aspects of pump operation are controlled by inputs from the cassette ID and piston latch detectors. If no cassette is installed, the mechanism is "homed" immediately. If a cassette is installed or partially installed homing is not performed, since this may result in unintentional fluid delivery to the patient. If a cassette has been properly installed, the system is ready for infusion to begin. If a cassette is not installed or latched, a prompt is given if starting the infusion is attempted.

If the pump is infusing, an open piston latch stops the infusion and produces audio and visual alarms. If the latch is closed again and START pressed, the infusion is started. If the cassette is not sensed, it is assumed that the cassette fell out, and the alarm continues (with a changed message) until it is cleared, which then causes the piston actuator to home.

### Fluid Pumping Control

The range of flow rates is from 0.1 to 999 ml/hr. The keep-vein-open (KVO) rate provides a small increment of fluid at a frequent enough interval to prevent a blood clot from forming on the catheter tip and occluding the line. The minimum time between pulses at minimum flow rate is 15 to 20 seconds. The 90% settling time for infusion rates in response to a step input command is 30 seconds or less. Any fluid delivery which is out of specification for a period of 120 seconds is detected by the system, which then alerts the user. When the pump is not being commanded to infuse, any delivery of fluid gives rise to an immediate alarm. The system detects any single-point sensor failure that would result in erroneous monitoring and control of fluid delivery. The system is failsafe in the event of a sensor failure.

### Position Sensing

The encoder 46 provides position feedback for control of the drive mechanism and cassette. The encoder provides a home position marker at 180° from top dead center for cassette loading. This marker is at the beginning of the fill stroke. A logic signal, DEL, is derived from the encoder that indicates the deliver and refill cycles. The DEL signal is logic one during the delivery cycle, which is from 0 to 120° from top dead center. During the refill cycle, which is from 120 to 360°, the DEL signal is logic zero. Incremental position markers are provided in the delivery cycle (0. to 120°) for precision feedback control of the fluid delivery rate.

### Direction Sensing

Direction sensing of the drive mechanism is necessary for reasons of safety and accuracy. The drive mechanism includes a mechanical brake which prevents all but a small amount of counter-rotation under mechanical loads and pulse operation. Failure of the anti-backdrive brake is detected by the system. Two quadrature incremental position tracks in the delivery cycle are used for detecting direction. A small amount of backdrive is always possible; this could result in inaccuracies in volume counting or fluid delivered.

### Resolution Requirements

The maximum time between pulses is 18 to 10 seconds. This is important to ensure a small trickle flow to keep the vascular access site patent. The time required to deliver one stroke of the cassette pulp is 3.6 Q/rate, where rate is in ml/hr and Q is the stroke volume in microlitres. Dividing by the maximum time desired between pulses yields the number of pulses required per delivery cycle. For example, for an 80 microlitre cassette and a rate of 0.1 ml/hr, if the maximum time between pulses is 18 seconds then 160 counts per delivery cycle are required.

### Delivery Cycle Incremental Position Counter

A quadrature counting circuit is included to count encoder pulses during the delivery cycle. The counter is reset at predetermined intervals by the microprocessor after the contents have been read. The output represents the change in angular position of the cam, which is related to the fluid delivery rate. Before it can begin counting, the counter must be enabled by the microprocessor. The counter is gated by the delivery cycle indicator signal, DEL, so that pulses are counted only during the delivery cycle.

### Brake Maker

At low rates where small increments of fluid are required, it is necessary to stop the motor at the end of the refill cycle to prevent over-infusion. Stopping the motor is accomplished more quickly by shorting the motor windings. This type of electronic braking makes use of the back EMF generated by the motor. Braking is not used for high infusion rates because overshoot is not a significant problem. The encoder provides a marker at a predetermined angle before the beginning of the delivery cycle that can be used for braking.

### Motor Actuation Circuit

Movement of the drive mechanism and cassette ( and subsequent movement of fluid) is controlled by the motor actuation circuit. The motor actuation circuit generates the voltage input to the motor and applied braking when needed. The voltage pulse generated is determined from the rate command, the output of the feedback controller, and the position sensing circuit.

At low rates, during the delivery cycle the drive voltage for the motor consists of a train of pulses. The period of the pulse train is a fixed interval set to 3 seconds and controlled by the microprocessor. At high rates the pulse width is greater than one cycle; i.e., multiple revolutions take place during one pulse width. The pulse width and amplitude are adjusted by the microprocessor to achieve the proper motor speed. The flow produced by the pump is proportional to the motor speed averaged over the pulse period.

The appropriate pulse width is determined by the microprocessor and loaded into the pulse generator circuit via the data bus. Once a pulse width is loaded, the microprocessor starts the pulse generator. The output of the pulse generator is logic high for the duration of the pulse width; otherwise it is logic zero. The pulse width is only measured during the delivery cycle; therefore, the pulse generator is gated by the delivery signal, DEL.

The range of pulse widths is from 1 millisecond to a second. The maximum is 1 second instead of 3 seconds because the delivery cycle is one-third of a revolution; therefore, one-third of the 3-second control period is the delivery cycle pulse width.

A steady-state speed of the motor is proportional to the amplitude of the drive voltage. To simplify the drive circuitry, only two pulse amplitudes are used (5 and 9 volts). Five volts is used for rate commands below 200 ml/hr; otherwise, 13 volts is used.

### Refill Cycle

Refilling must be performed as fast as possible in order to minimise the time during which fluid is not delivered to the patient. Refilling is accomplished by loading a pulse width into the pulse generator and starting the motor. The pulse generator is gated by the DEL signal so that no counting takes place during the refill cycle. Refill uses the currently selected drive voltage (5 or 13 volts). Electronic braking is used for flow rates less than a certain set amount. The control logic performs this function when the brake is enabled by the microprocessor.

### Homing

When commanded by the microprocessor to home, the motor voltage is set to 13 volts. When the home position marker on the encoder is detected, the electronic brake is applied. The cam must be positioned sufficiently close to the home position so that fluid will not be delivered to the patient.

### Power Considerations in Multichannel Pump Operation

In multichannel operation, the leading edges of the pulses for the motors are staggered in order to prevent an excessive peak current drain from the power supply. A transient current increase occurs in the motor circuit when a step increase in voltage is applied. The peak current is approximately the supply voltage divided by the armature resistance, which occurs at the onset of voltage. The current exponentially decays from this value with a time constant of 10 to 20 milliseconds. In the period equivalent to four time constants the transient will have decayed 98%, corresponding to 40 to 80 milliseconds.

### Feedback Control of Delivery Rate

Closed-loop feedback control of motor speed (and hence fluid delivery rate) compensates for changes in motor and load characteristics. The input of the control system is the rate command. The output of the system is fluid flow, but since this is not directly observable, an indirect method is used for obtaining the feedback signal. Flow is estimated in the microprocessor from the average rotational speed of the drive, calculated using data from the encoder about delivery cycle incremental position. The difference between the rate command and estimated rate is the error signal. The feedback controller attempts to drive the error signal to zero by adjusting the pulse width using a nonlinear proportional-plus-integral-plus derivative (nPID) algorithm.

### Rate Estimation

The average fluid delivery rate is estimated using data from the position counter during the delivery cycle. The microprocessor periodically samples the counter to determine the amount of fluid delivered. The sampling period was selected as 3 seconds based on the desired settling time, the need to provide frequent pulsing for rate accuracy and KVO requirements, minimising the number of calculations required (which minimises power), and minimising motor starting and stopping (which adversely affects motor life). Using a single sampling period for all rates also simplifies the software. Since 160 encoder pulses are produced over an 80-microlitre delivery stroke, each encoder pulse represents delivery of 0.5 microlitre of fluid.

### Feedback Controller

A digital feedback controller determines the motor pulse width required to maintain the estimated rate at the rate command. The input to the controller is the sampled error signal, e, which is the difference between the rate command, r_{c}, and the estimated rate rₑ. At predetermined intervals, the controller (by means of a digital computer program) adjusts the pulse width based on the error signal. The calculated pulse width count is then transferred to the motor pulse width generator. The initial pulse width value is determined from open-loop data; this is used to decrease the settling time.

### Timing Requirements

A proper sequence of events in the controller is necessary to ensure closed-loop stability and accuracy. For each pumping channel, the sequence performed at 3-second intervals (the controller sampling period) is given below. This sequence is performed only if the infusion is started and there are no faults detected.

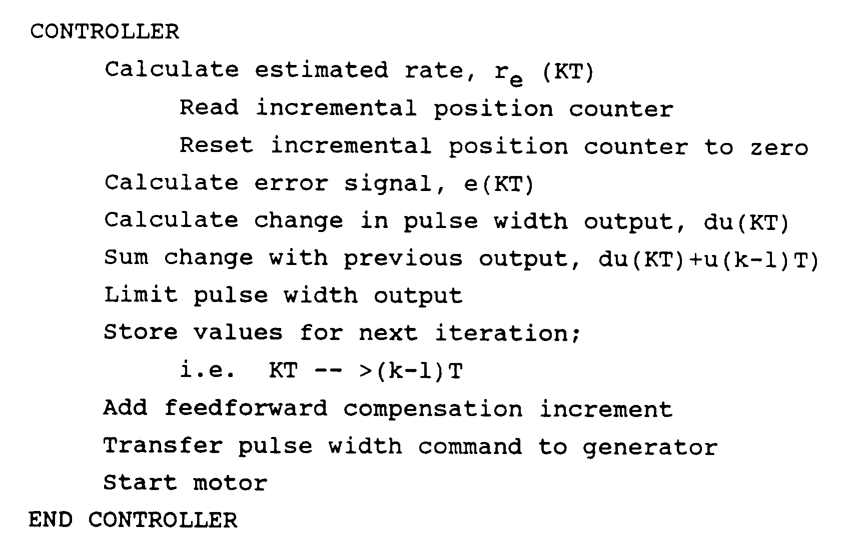

### Software Implementation of Motor Control

As shown in Figure 6, three distinct software components, the infusion control task 108, the pump control timer 110, and the motor control task 112 generate controlled fluid delivery to the three motors in the embodiment depicted.

The infusion control task (ICT) co-ordinates all aspects of fluid delivery to the three pumps. This software block is told when to start or stop an infusion, and it responds accordingly. It also monitors the course of the infusion and informs the system of any alarms (air in line, occlusion, etc.) or when the infusion is complete. The ICT does not directly control the motors; it merely decides when each motor should begin and end delivery of fluid, or when the infusion rate should change. When an infusion is to begin, stop, or change rate, the ICT sends a message to either the pump control timer or the motor control task to perform the actual details. The ICT keeps track of the state of each pump by maintaining a state variable. This state variable can have the values "READY", "HOMING", "INFUSING", etc.

The pump control timer includes a procedure executed at regular intervals, performing various monitoring functions for the ICT and MCT. The PCT also generates the messages to the motor control task at three-second intervals which cause closed-loop control of each pump to occur. The PCT executes every 60 milliseconds. This interval provides the basic clock that controls all motor control timing. The choice of 60 milliseconds was influenced by several considerations. Part of the job of the PCT is to monitor the DEL signal from the encoder. Assuming a maximum motor speed of five revolutions per second, a delivery cycle can occur in 67 milliseconds. Therefore, the timer must sample the DEL signal at least this frequently. The control interval for closed-loop motor control was chosen to be three seconds. Since the PCT provides the basic timing for the motor control task, the PCT must execute at a frequency which is a factor of three seconds, i.e. 3000 milliseconds must be an integral multiple of the timing frequency of the pump timer. To reduce peak current demands, the starting of each motor must be staggered so that the commands to start two motors are no closer together than 25 milliseconds. The operating system clock operates at an interval of 20 milliseconds, so that all timer routines must be an integral multiple of 20 milliseconds. Finally, the timer must operate at as low a frequency as possible to reduce the workload on the microprocessor and thereby reduce power consumption.

Every 60 milliseconds, the instrument operating system causes the PCT to execute. The PCT routine performs two types of functions: 1) general motor position dependent monitoring of all three pumps. This requires checking the DEL signal on each pump to determine whether each pump is in a fill or delivery cycle. Appropriate functions (unrelated to motor control timing) such as encoder checks and VR/VI/TVI (veinous rate/veinous infusion/total veinous infusion) updating are then performed. The timer then determines which of the pumps requires more handing. At each 60 millisecond execution, the timer routine goes through the next step in a five-part cycle. Steps 1 through 3 cause special processing to be performed for pumps a, b, and c. Steps 4 and 5 are no operations (no further processing is performed by the PCT). Figure 7 shows the PCT timing.

Since the special processing for each pump occurs only in one of five pump control executions, each pump processing occurs at 300 millisecond intervals and the processing between pumps is at least 60 milliseconds. This 60 millisecond gap between pump processing prevents pumps from starting at the same instant. The special processing that occurs every 300 milliseconds for each pump consists of checking the state variable for the pump, monitoring appropriate signals, and generating messages to the ICT and/or MCT where needed.

The motor control task (MCT) handles the job of running the motors so that fluid is delivered at a prescribed rate. The MCT can be instructed to perform various functions by reception of the following messages: a) "perform open-loop control on pump X"--the MCT responds to this instruction by sending one pulse to the motor at a "best guess" voltage and pulse width, so that pump X will deliver fluid at approximately the prescribed rate for the following three seconds. This message is used to initiate fluid delivery or change rates on pump X. b) "perform closed-loop control on pump X"--the MCT is given an estimate of how fast the motor has been running over the last three seconds. The MCT uses this information to determine the size of the next pulse to put out to the motor to maintain the prescribed fluid rate. c) "stop pump X"--the MCT sets up the motor control software so that the motor is stopped. d) "home pump X"--the PCT generates the signals necessary to cause the motor to move to the home position.

### Optional Sequences

The following sequence of events occurs in response to various infusion control commands:
Start infusion on pump X--see Figure 8. The ICT sets the state of the motor to START RUN. At the next execution of the PCT at which the pump control processing for pump X is performed, a "perform open-loop control on pump X" message is sent to the MCT. The state variable is set to RUNNING. The PCT sets up a counter so that every three seconds the estimated rate of the motor for pump X is calculated and a "perform closed-loop on pump X" message to the MCT is generated.

Stop infusion on pump X--the ICT sends a "stop pump X" message to the MCT. The ICT sets the state of pump X to STOPPING so that the PCT does not generate any more "perform closed-loop control on pump X" messages.

Change rate on pump X--see Figure 9. The ICT sends a "perform open-loop control on pump X" message directly to the MCT. After the MCT executes the open-loop control procedure, the motor for pump X will run open-loop until the MCT receives the next message from the PCT directing the MCT to perform closed-loop control.

Home pump X--the ICT sends a "home pump X" message directly to the MCT and sets the state of pump X to HOMING. The MCT begins the homing sequence. The PCT, noting that pump X state is homing, will watch the motor position. When the motor reaches home, the pump state variable is set to NO SET (homing is only performed when no cassette is in place).

Although there have been described above specific arrangements of a fluid delivery control and monitoring apparatus for a medication infusion system for the purpose of illustrating the manner in which the invention may be used to advantage, it will be appreciated that the invention is not limited thereto.

## Claims

1. Fluid delivery, monitoring and control apparatus for use in a medication infusion system, employing a disposable fluid pathway, the apparatus comprising: drive means (14) for actuating pistons and valves in the fluid pathway and monitoring means (16), having inputs (20) from sensors in the drive means; control means (18) having inputs from the monitoring means, from the drive means, and from command signals (24); and an output connected to control the drive means; and encoding means (46) coupled to the drive means and arranged to provide signals indicative of the position of the drive means; characterised in that the pistons and valves are mounted in a disposable cassette (12), the monitoring means also having inputs (20) from sensors in the cassette; the encoding means are also arranged to provide rate of movement signals from the drive means; the monitoring means include means for ascertaining fluid flow rate from the rate of movement signals and from cassette indicia, indicating piston stroke volume, and for generating feedback signals indicative of sensed flow rate; and in that the control means include means for receiving rate command signals (24), defining a desired rate of fluid flow through an associated cassette, and means for combining the rate command signals with the feedback signals to develop signals for controlling the drive means.

2. Apparatus as claimed in Claim 1, characterised in that the drive means comprise at least one drive mechanism (14), each mechanism comprising; a bottom support structure; a motor mounted on the support structure with gears (36) attached to a drive shaft of the motor, there being an output shaft on one of the gears; a cam (38) coupled to be driven by the output shaft; an inlet valve actuator (40) mechanically coupled to an inlet valve (28) forming part of a pump in the cassette, the inlet valve actuator being driven by the cam; a piston actuator (42) mechanically coupled to a piston (30) forming part of the pump in the cassette, the piston actuator being driven by the cam; an outlet valve actuator (44) mechanically coupled to an outlet valve (32) forming part of the pump in the cassette, the outlet valve actuator being driven by the cam; the encoder means (46) being arranged on the support structure and connected to the output shaft to provide signals indicative of the orientation of the cam.

3. Apparatus as claimed in Claim 2, characterised in that the control means further include one or more motor control means, each comprising; a feedback controller (60) responsive to input commands (64) and alarm signals (68), having a position sensing input (62) and a control output; motor actuation circuitry (58) controlled by the output of the feedback controller; and a position sensing circuit (50) having an input from the encoder means and an output (62) connected to the feedback controller.

4. Apparatus as claimed in Claim 3, characterised in that the or each feedback controller is a microprocessor (70) which comprises a nonlinear controller (74) having an error signal input (e) and an output; a rate estimator (72) having a position counter input, a cassette/volume input, and an estimated rate output; a subtractor (102) having a first input connected to the output of the rate estimator; a second, rate command input (104), and an error output (e) connected to the input of the nonlinear controller; a motor supply voltage circuit (76), having a motor voltage output determined by an input (106) connected in parallel with the rate command input; and a control logic input signal generator for producing counter reset (25), counter enable (86), motor actuation circuitry start (88) and motor brake enable (90) signals.

5. Apparatus as claimed in Claim 4, characterised in that the motor actuation circuitry comprises; a pulse generator (78) having an input from the nonlinear controller and an output; a motor driver (80), having a first input from the motor voltage supply circuit, a second input from the pulse generator, and a third input arranged to effect a braking action on the motor; and a control logic unit (82) operable to provide start and gate signals (83,84) to the pulse generator and reset (94), enable (96) and gate (98) signals to the position counter, and a brake signal to the motor driver, the unit having input lines from the encoder means and from the control logic signal generator.

6. Apparatus as claimed in Claim 4 or 5, characterised in that the position sensing circuit comprises a position counter (92) having an input (100) from the encoder means and an output to the rate estimator.

7. Apparatus as claimed in Claim 4, 5 or 6, characterised in that the microprocessor is operable to perform an infusion control task (ICT) which co-ordinates fluid delivery by the plurality of pumps; a motor control task (MCT) which runs the plurality of motors to deliver fluid at a prescribed rate; and a pump control timer (PCT) which monitors functions of the ICT and MCT and also generates messages for the MCT which cause closed-loop control of the plurality of pumps.

8. Apparatus as claimed in Claim 5 or 6 or 7, characterised in that the controller comprises digital computer means programmed to adjust a pulse width of the output of the pulse generator based on the error sign entering the error input, the programme consisting of a sampled-data nonlinear proportional-plus-integral-plus-derivative (PID) algorithm.

9. Apparatus as claimed in any of Claims 2 to 8, characterised in that each motor comprises an anti-backdrive clutch arrangement for braking.

10. Apparatus as claimed in any of Claims 2 to 9, characterised in that the encoder means comprise; an encoder disc (48) mounted on the output shaft, the disc having a plurality of fiducial indicia (55) on a face thereof corresponding to a "home" position, and defining segments corresponding to a delivery cycle, a valve-to-fill cycle, a fill stroke, a valve-to-deliver cycle and a predetermined time (56) before the beginning of the delivery cycle for braking; and means responsive to the indicia for producing electrical signals as the disc rotates.

11. Apparatus as claimed in Claim 10, characterised in that the means for producing electrical signals comprise a photoelectric detector used to detect light from the face of the encoder disc to generate electrical signals correlated with positions of the indicia on the face of the encoder disc.

## Patentansprüche

1. Vorrichtung zum Zuführen, Überwachen und Steuern von Fluiden zur Verwendung in einem medizinischen Infusionssystem, bei der ein austauschbarer Strömungsweg für das Fluid eingesetzt wird, wobei die Vorrichtung folgende Merkmale aufweist:
Antriebsmittel (14) zur Betätigung von Kolben und Ventilen im Strömungsweg des Fluids und Überwachungsmittel (16) mit Eingängen (20) für Sensoren in den Antriebsmitteln,
Steuermittel (18) mit Eingängen für die Überwachungsmittel, für die Antriebsmittel und für Befehlsignale (24) und mit einem zur Steuerung der Antriebsmittel angeschlossenen Ausgang,
an die Antriebsmittel angekappelte Codiermittel (46), die so angeordnet sind, daß sie Signale entsprechend der Position der Antriebsmittel erzeugen,
**dadurch gekennzeichnet**
daß die Kolben und Ventile in einer Einwegkassette (12) angeordnet sind und die Überwachungsmittel auch Eingänge (20) für Sensoren in der Kassette haben,
daß die Codiermittel so ausgebildet sind, daß sie auch Signale entsprechend dem Bewegungszustand der Antriebsmittel erzeugen,
daß die Überwachungsmittel Einrichtungen zur Ermittlung des Strömungsdurchsatzes des Fluids aus den Signalen entsprechend dem Bewegungszustand und aus das Kolbenhubvolumen angebenden Anzeigen der Kassette sowie zur Erzeugung von den festgestellten Strömungsdurchsatz angebenden Rückkopplungssignalen aufweisen,
und daß die Steuermittel Mittel zur Eingabe von Durchsatzsteuersignalen (24), welche den gewünschten Durchsatz des Fluidstroms durch eine zugehörige Kassette festlegen, und Mittel aufweisen, um die Durchsatzsteuersignale mit den Rückkopplungssignalen so zu verbinden, daß Signale zum Steuern der Antriebsmittel erzeugt werden.

2. Vorrichtung nach Anspruch 1, dadurch gekennzeichnet, daß die Antriebsmittel mindestens einen Antriebsmechanismus (14) umfassen, wobei bei jedem Mechanismus die folgenden Merkmale vorhanden sind:
- eine Tragstruktur am Boden;
- ein auf der Tragstruktur befestigter Motor mit auf einer Antriebswelle des Motors befestigten Zahnrädern (36), wobei sich an einem der Zahnräder eine Abtriebswelle befindet;
- eine Nocke (38), die so angekoppelt ist, daß sie von der Abtriebswelle angetrieben wird;
- ein Einlaßventil-Betätigungselement (40), das mechanisch an ein einen Bestandteil einer Pumpe in der Kassette bildendes Einlaßventil (28) angekoppelt ist und durch die Nocke angetrieben wird;
- ein Kolbenbetätigungselement (42), das mechanisch an einen einen Bestandteil der Pumpe in der Kassette bildenden Kolben (30) angekoppelt ist und von der Nocke angetrieben wird;
- ein Auslaßventil-Betätigungselement (44), das mechanisch an ein einen Bestandteil der Pumpe in der Kassette bildendes Auslaßventil (32) angekoppelt ist und durch die Nocke angetrieben wird;
- die Codiermittel (46) sind auf der Tragstruktur angeordnet und an die Abtriebswelle angeschlossen, um Signale entsprechend der Nockenorientierung zu erzeugen.

3. Vorrichtung nach Anspruch 2, dadurch gekennzeichnet, daß die Steuermittel ferner eine oder mehrere Motorsteuereinrichtungen aufweisen, von denen jede die folgenden Merkmale enthält:
- eine rückgekoppelte Steuereinheit (60), die auf Eingangsbefehle (64) und Alarmsignale (68) anspricht und einen positionserfassenden Eingang (62) und einen Steuerausgang aufweist,
- einen Motorbetreibungsschaltkreis (58), der vom Ausgang der rückgekoppelten Steuereinheit aus gesteuert wird,
- und einen positionserfassenden Schaltkreis (50) mit einem an die Codiermittel angeschlossenen Eingang und einem an die rückgekoppelte Steuereinheit angeschlossenen Ausgang (62).

4. Vorrichtung nach Anspruch 3, dadurch gekennzeichnet, daß die oder jede rückgekoppelte Steuereinheit ein Mikroprozessor (70) ist, der eine nichtlineare Steuerung (74) mit einem Fehlersignaleingang (e) und einen Ausgang,
eine Durchsatzabschätzeinrichtung (72) mit einem Positionszähleingang, einem Kassetten/Volumeneingang und einem Ausgang für den geschätzten Durchsatz, einen Subtrahierer (102) mit einem ersten Eingang, der mit dem Ausgang der Durchsatzabschätzeinrichtung verbunden ist, mit einem zweiten Eingang als Durchsatzsteuereingang (104) und mit einem Fehlersignalausgang (e), der mit dem Eingang der nichtlinearen Steuerung verbunden ist,
eine Schaltung (74) zur Spannungsversorgung des Motors (76), die eine Motorspannung in Abhängigkeit einer Spannung an einem Eingang (106) abgibt, der parallel mit dem Durchsatzsteuereingang geschaltet ist,
und einen Signalgenerator für die Signaleingabe in die Steuerlogik zur Erzeugung eines Zählerrücksetzsignals (25), eines Zählerfreigabesignals (86), eines Startsignals (88) für den Motorbetreibungsschaltkreis und eines Motorbremsfreigabesignals (90) enthält.

5. Vorrichtung nach Anspruch 4, dadurch gekennzeichnet, daß der Motorbetreibungsschaltkreis folgende Merkmale aufweist:
- einen Pulsgenerator (78) mit einem an der nichtlinearen Steuereinheit angeschlossenen Eingang und einem Ausgang;
- einen Motortreiber (80) mit einem ersten an die Schaltung zur der Spannungsversorgung des Motors angeschlossenen Eingang, einem zweiten am Pulsgenerator angeschlossenen Eingang und einem dritten Eingang, der dazu eingerichtet ist, einen Bremsvorgang am Motor zu bewirken, und
- eine Steuerlogikeinheit (82) zur Erzeugung von Start- und Sperrsignalen (83, 84) für den Pulsgenerator und von Rücksetzsignalen (94), Freigabesignalen (96) und Sperrsignalen (98) für den Positionszähler sowie eines Bremssignals für den Motortreiber, wobei die Einheit Eingänge hat, die von den Codiermitteln und vom Signalgenerator für die Steuerlogik gespeist sind.

6. Vorrichtung nach Anspruch 4 oder 5, dadurch gekennzeichnet, daß der positionserfassende Schaltkreis einen Positionszähler (92) mit einem an die Codiermittel angeschlossenen Eingang (100) und einem an die Durchsatzabschätzeinrichtung angeschlossenen Ausgang aufweist.

7. Vorrichtung nach Anspruch 4, 5 oder 6, dadurch gekennzeichnet, daß der Mikroprozessor zur Ausführung der folgenden Programme betreibbar ist:
eine Infusionssteuertask (ICT), welche die Zuführung des Fluids durch die Mehrzahl von Pumpen koordiniert, eine Motorsteuertask (MCT), welche die Mehrzahl von Motoren zur Zuführung des Fluids mit einem vorgeschriebenen Durchsatz betreibt, und einen Zeitgeber (PCT) für die Pumpensteuerung, der die Funktionen der ICT und MCT überwacht und auch Meldungen für die MCT erzeugt, wodurch für die mehreren Pumpen eine Steuerung mit einer geschlossenen Schleife gegeben ist.

8. Vorrichtung nach Anspruch 5, 6 oder 7, dadurch gekennzeichnet, daß die Steuerung digitale Computermittel enthält, die zur Einstellung einer Pulsbreite des Ausgangssignals des Pulsgenerator in Abhängigkeit von Fehlervorzeichen am Fehlereingang programmiert ist, wobei das Programm einen nichtlinearen Proportional/Integral/Ableitungs- (PID) Algorithmus für die aufgenommenen Daten enthält.

9. Vorrichtung nach einem der Ansprüche 2 bis 8, dadurch gekennzeichnet, daß jeder Motor eine Kupplungsanordnung zum Verhindern eines Rücklaufs beim Bremsen enthält.

10. Vorrichtung nach einem der Ansprüche 2 bis 9, dadurch gekennzeichnet, daß die Codiermittel
eine auf der Ausgangswelle befestigte Codierscheibe (48), die eine Mehrzahl von Bezugsmarken (55) auf einer ihrer Flächen hat, die einer Nullposition entsprechen und Segmente bestimmen, die einem Zuführzyklus, einem Ventilfüllzyklus, einem Füllhub, einem Ventilzuführzyklus und einer vorbestimmten Zeit (56) vor dem Start des Zuführzyklus zum Bremsen entsprechen,
und Einrichtungen aufweisen, die auf die Marken beim Drehen der Scheibe zum Erzeugen elektrischer Signale ansprechen.

11. Vorrichtung nach Anspruch 10, dadurch gekennzeichnet, daß die Mittel zum Erzeugen elektrischer Signale einen photoelektrischen Detektor zum Erfassen von Licht von der Oberfläche der Codierscheibe enthalten, um elektrische Signale entsprechend den Positionen der Marken auf der Fläche der Codierscheibe zu erzeugen.

## Revendications

1. Dispositif de commande, contrôle et délivrance de fluide utilisable dans un système d'infusion de médicament, employant un passage de fluide jetable, le dispositif comprenant : un moyen d'entraînement (14) pour actionner des pistons et des soupapes situés dans le trajet de fluide ainsi qu'un moyen de contrôle (16), comportant des entrées (20) reliées à des capteurs du moyen d'entraînement ; un moyen de commande (18) comportant des entrées reliées au moyen de contrôle, au moyen d'entraînement et recevant des signaux de commande (24) ; et une sortie connectée de façon à commander le moyen d'entraînement ; et un moyen de codage (46) relié au moyen d'entraînement et agencé pour produire des signaux représentant la position du moyen d'entraînement ; caractérisé en ce que les pistons et les soupapes sont montés dans une cassette jetable (12), le moyen de contrôle comportant également des entrées (20) reliées à des capteurs situés dans la cassette ; le moyen de codage étant également agencé pour produire des signaux représentant la vitesse de déplacement du moyen d'entraînement; le moyen de contrôle comprenant des moyens pour déterminer un débit de fluide à partir des signaux représentant une vitesse de déplacement et à partir de repères prévus sur la cassette et indiquant des volumes par courses de pistons, et pour produire des signaux de réaction représentant un débit détecté; et en ce que le moyen de commande comprend des moyens pour recevoir des signaux de commande de débit (24), définissant un débit désiré d'écoulement de fluide dans une cassette associée, ainsi que des moyens pour combiner les signaux de commande de débit avec les signaux de réaction afin de produire des signaux servant à la commande du moyen d'entraînement.

2. Dispositif tel que revendiqué dans la revendication 1, caractérisé en ce que le moyen d'entraînement comprend au moins un mécanisme d'entraînement (14), chaque mécanisme comprenant : une structure portante de base ; un moteur monté sur la structure portante, avec des pignons (36) fixés sur un arbre d'entraînement du moteur, un arbre de sortie étant prévu sur un des pignons ; une came (38) accouplée de façon à être entraînée par l'arbre de sortie ; un organe (40) d'actionnement de soupape d'entrée accouplé mécaniquement à une soupape d'entrée (28) faisant partie d'une pompe située dans la cassette, l'organe d'actionnement de la soupape d'entrée étant entraîné par la came; un organe (42) d'actionnement de piston accouplé mécaniquement à un piston (30) faisant partie de la pompe située dans la cassette, l'organe d'actionnement de piston étant entraîné par la came ; un organe (44) d'actionnement de soupape de sortie accouplé mécaniquement avec une soupape de sortie (32) faisant partie de la pompe située dans la cassette, l'organe d'actionnement de la soupape de sortie étant entraîné par la came ; le moyen de codage (46) étant disposé sur la structure portante et étant relié à l'arbre de sortie de façon à produire des signaux indiquant l'orientation de la came.

3. Dispositif tel que revendiqué dans la revendication 2, caractérisé en ce que le moyen de commande comprend en outre un ou plusieurs éléments de commande de moteur, comprenant chacun : une commande corrective (60) répondant à des ordres d'entrée (64) et des signaux d'alarme (68) et comportant une entrée (62) de détection de position et une sortie de commande ; un circuit d'actionnement de moteur (58) commandé par le signal de sortie de la commande corrective ; et un circuit de détection de position (50) comportant une entrée reliée au moyen de codage et une sortie (62) reliée à la commande corrective.

4. Dispositif tel que revendiqué dans la revendication 3, caractérisé en ce que la ou chaque commande corrective est un microprocesseur (70) qui comprend une commande non linéaire (74) comportant une entrée (e) de signaux d'erreurs et une sortie ; un moyen d'estimation de débit (72) comportant une entrée reliée au compteur de position, une entrée de cassette/volume et une sortie de débit estimé ; un soustracteur (102) comportant une première entrée reliée à la sortie du moyen d'estimation de débit ; une seconde entrée (104) d'ordres de débit et une sortie d'erreurs (e) reliée à l'entrée de la commande non linéaire; un circuit (76) de génération de tension d'alimentation pour le moteur, comportant une sortie de tension de moteur déterminée par une entrée (106) reliée en parallèle à l'entrée d'ordre de débit ; et un générateur de signaux d'entrée à la logique de commande pour produire des signaux de remise à zéro de compteur (25), de validation de compteur (86), d'enclenchement de circuit d'actionnement de moteur (88) et de validation de freinage de moteur (90).

5. Dispositif tel que revendiqué dans la revendication 4, caractérisé en ce que le circuit d'actionnement de moteur comprend : un générateur d'impulsions (78) comportant une entrée reliée à la commande non linéaire et une sortie ; un pilote de moteur (80), comportant une première entrée reliée au circuit d'alimentation en tension de moteur, une seconde entrée reliée au générateur d'impulsions et une troisième entrée agencée pour produire une action de freinage sur le moteur ; et une unité logique de commande (82) pouvant opérer pour appliquer des signaux d'enclenchement et de synchronisation (83,84) au générateur d'impulsions et des signaux de remise à zéro (94), de validation (96) et de synchronisation (98) au compteur de position, ainsi qu'un signal de freinage au pilote de moteur, l'unité comportant des lignes d'entrée partant du moyen de codage et partant également du générateur de signaux pour la logique de commande.

6. Dispositif tel que revendiqué dans la revendication 4 ou 5, caractérisé en ce que le circuit de détection de position comprend un compteur de position (92) comportant une entrée (100) reliée au codeur et une sortie reliée au moyen d'estimation de débit.

7. Dispositif tel que revendiqué dans la revendication 4, 5 ou 6, caractérisé en ce que le microprocesseur peut opérer pour effectuer une tâche de commande d'infusion (ICT) qui coordonne une délivrance de fluide par des différentes pompes; une tâche de commande de moteur (MCT) qui fait fonctionner les différents moteurs pour délivrer du fluide à un débit prescrit ; et une minuterie de commande de pompe (PCT) qui contrôle les fonctions des tâches ICT et MCT et qui produit également des messages pour la tâche MCT qui assure une commande en boucle fermée des différentes pompes.

8. Dispositif tel que revendiqué dans la revendication 5 ou 6 ou 7, caractérisé en ce que le moyen de commande comprend un ordinateur numérique programmé de façon à régler une largeur d'impulsion du signal de sortie du générateur d'impulsions sur la base du signe d'erreur introduit dans l'entrée d'erreur, le programme comprenant un algorithme non linéaire pour données échantillonnées du type PID (proportionnalité-plus-intégration-plus-dérivation).

9. Dispositif tel que revendiqué dans une quelconque des revendications 2 à 8, caractérisé en ce que chaque moteur comprend un dispositif d'accouplement anti-retour pour le freinage.

10. Dispositif tel que revendiqué dans une quelconque des revendications 2 à 9, caractérisé en ce que le moyen de codage comprend : un disque de codage (48) monté sur l'arbre de sortie, ce disque comportant plusieurs repères de référence (55) sur une face en correspondance à une position "repos", et définissant des segments correspondant à des cycles de commande de délivrance, de soupape de remplissage, de charge de remplissage, de soupape de délivrance et d'un temps prédéterminé (56) avant le début du cycle de remplissage en vue d'un freinage ; et des moyens répondant aux indices pour produire des signaux électriques quand le disque tourne.

11. Dispositif tel que revendiqué dans la revendication 10, caractérisé en ce que les moyens de génération de signaux électriques comprennent un détecteur photoélectrique utilisé pour détecter de la lumière provenant de la face du disque de codage afin de produire des signaux électriques en corrélation avec des positions des indices situés sur la face du disque de codage.
